# EUROPÄISCHE PATENTSCHRIFT

(11) **EP 0 946 578 B1**
(45) Veröffentlichungstag und Bekanntmachung des Hinweises auf die Patenterteilung: **16.03.2005**
(21) Anmeldenummer: 97952917.9
(22) Anmeldetag: 11.12.1997
(51) Int. Cl.: C07H 15/203, B01J 31/24

(54) **PHOSPHANE, VERFAHREN ZU IHRER HERSTELLUNG UND IHRE VERWENDUNG IN METALLKOMPLEXEN**
PHOSPHANES, THEIR METHOD OF PRODUCTION AND USE IN METAL COMPLEXES
PHOSPHANES, LEUR PROCEDE DE PRODUCTION ET LEUR UTILISATION DANS DES COMPLEXES METALLIQUES

(30) Priorität: 23.12.1996 DE 19654180
(43) Veröffentlichungstag der Anmeldung: 06.10.1999
(73) Patentinhaber: Celanese Chemicals Europe GmbH, 60439 Frankfurt am Main (DE)
(72) Erfinder: BOGDANOVIC, Sandra, D-60322 Frankfurt (DE); BELLER, Matthias, D-85737 Ismaning (DE); ZAPF, Alexander, D-83024 Rosenheim (DE); KRAUTER, Jürgen, D-86161 Augsburg (DE)
(86) Internationale Anmeldenummer: PCT/EP1997/006931
(87) Internationale Veröffentlichungsnummer: WO 1998/028315

(56) Entgegenhaltungen:
- EP-A- 0 704 449
- O.NEUNHOFFER ET AL.: "Diphenyl-(p-hydroxy-phenyl)-phosphin-B-D- Glucosid." CHEMISCHE BERICHTE, Bd. 94, Nr. 9, 1961, Seiten 2519-2521, XP002058429
- D.DESS ET AL.: "Phase-Transfer Catalyzed Synthesis of Acetylated Aryl B-D-Glucopyranosides and Aryl-B-D-Galactopyranosides." SYNTHESIS, Nr. 11, November 1981, STUTTGART DE, Seiten 883-885, XP002058430
- R.ROY ET AL.: "Carbohydrate Protein Interactions. Syntheses of Agglutination Inhibitors of Wheat Germ Agglutinin by Phase Transfer Catalysis." CANADIAN JOURNAL OF CHEMISTRY., Bd. 69, Nr. 5, Mai 1991, OTTAWA CA, Seiten 817-821, XP002058431
- M.BELLER ET AL.: "Carbohydrate-Substituted Triarylphosphanes - A New Class of Ligands for Two-Phase Catalysis." ANGEWANDTE CHEMIE. INTERNATIONAL EDITION., Bd. 36, Nr. 7, 18.April 1997, WEINHEIM DE, Seiten 772-774, XP002058432

## Beschreibung

Die Erfindung betrifft hydrophile Phosphane, Verfahren zu ihrer Herstellung, ihre Verwendung in Metallkomplexen, diese Metallkomplexe und deren Verwendung in katalytischen Umsetzungen.

Viele chemische Umsetzungen von organischen Verbindungen werden unter homogener Katalyse durchgeführt. Ein generelles Problem der homogenen Katalyse ist dabei die einfache und ökonomische Abtrennung des Katalysatorsystems von den organischen Produkten nach der Umsetzung. Der Katalysator sollte vorteilhafterweise einfach von den organischen Produkten abzutrennen und in die Umsetzung rückführbar sein. Eine Lösung für diese Probleme bietet die sogenannte Zweiphasenkatalyse, bei der das Katalysatorsystem und die organischen Produkte in verschiedenen Phasen vorliegen und durch einfaches Dekantieren voneinander getrennt werden können. Solche zweiphasenkatalysierten Umsetzungen sind beispielsweise beschrieben in W.A. Herrmann, C.W. Kohlpaintner, Angew. Chem. 1993, 105, 1588-1609 und in P. Kalck, F. Monteil, Adv. Organomet. Chem. 1992, 34, 219. Häufig wird dabei als Katalysatorphase eine hydrophile Wasserphase eingesetzt, es können jedoch auch fluorierte Kohlenwasserstoffe oder Polyethylenglykole verwendet werden. Voraussetzung für eine effektive Zweiphasenkatalyse ist eine hohe Löslichkeit des Katalysatorsystems in der Katalysatorphase und eine geringe Löslichkeit in der Produktphase. Hierzu wurden in der Vergangenheit bei der Verwendung von Metallkomplexkatalysatoren hauptsächlich hydrophil modifizierte Liganden eingesetzt. Ein typisches Beispiel dafür sind sulfonierte Triarylphosphane, wie sie beispielsweise in W.A. Herrmann, C.W. Kohlpaintner, Angew. Chem. 1993, 105, 1588-1609 und in P. Kalck, F. Monteil, Adv.

Organomet. Chem. 1992, 34, 219 beschrieben sind. Sie werden technisch zur Hydroformylierung von Propen eingesetzt. Bis heute wurden im wesentlichen ionische Gruppen wie -SO₃⁻; -CO₂⁻, -NR₃⁺,- P(O)O₂²⁻ zur Erhöhung der Hydrophilie von Phosphanliganden eingesetzt. Dies ist beispielsweise beschrieben in M. Beller, B. Cornils, C.D. Frohning, C.W. Kohlpaintner, J. Mol. Catal. 1995, 104, 17-85. Ionische Funktionalitäten können aufgrund der vorhandenen Salzkonzentrationen in verschiedenen Verfahren nachteilig sein. Darüber hinaus sind keine chiralen ionischen Gruppen zugänglich. Zur Vermeidung dieser Nachteile können neutrale Substituenten eingesetzt werden. Als neutrale Substituenten zur Erhöhung der Hydrophilie von Phosphanen wurden bislang beispielsweise Polyethylenglykolgruppen verwendet. Dies ist beispielsweise in B. Cornils, Angew. Chem. 1995, 107, 1709-1711 beschrieben.

Aufgabe der vorliegenden Erfindung ist die Bereitstellung von hydrophilen Phosphanverbindungen, die als Metallkomplexliganden zur Herstellung von Katalysatoren verwendet werden können und eine Vielzahl von Strukturvarianten einschließlich chiraler Zentren zugänglich machen.

Diese Aufgabe wird gelöst durch ein Verfahren zur Herstellung von Phosphanen der allgemeinen Formel (I)

Ar¹ ₃₋ₓP(Ar²(OZ)_{y})ₓ (I)

in der Ar¹ und Ar² unabhängig gegebenenfalls mit Halogenatomen, Carboxylgruppen, Hydroxylgruppen, C₁₋₆-Alkylgruppen, Phenylgruppen oder Naphthylgruppen substituierte aromatische C₆₋₂₀-Reste oder C₄₋₉heteroaromatische C₄₋₉-Reste sind, wobei Ar¹ eine und Ar^{z} y + 1 freie Valenzen besitzen,
Z ein glycosidisch gebundener Kohlenhydratrest ist,
x den Wert 1, 2 oder 3 und
y den Wert 1 oder 2 hat,
durch
(1) Umsetzung von Kohlenhydrathalogenosen der allgemeinen Formel (II)

   Z'- X (II)

   in der Z' ein wie vorstehend angegebener Rest Z ist, in dem die Hydroxylgruppen durch Schutzgruppen substituiert sind, und X Br, Cl, oder F ist,
   mit einem Phosphan der allgemeinen Formel (III)

   Ar¹ ₃₋ₓP(Ar²(OH)_{y})ₓ

   in der Ar¹, Ar², x, y die vorstehende Bedeutung haben, in einem mehrphasigen Reaktionsmedium in Gegenwart einer Base und eines Phasentransferkatalysators,
   zu einem Phosphan der allgemeinen Formel (IV)

   Ar¹ ₃₋ₓP(Ar²(OZ')_{y})ₓ (IV)

   in der Ar¹, Ar², x, y und Z' die vorstehend angegebene Bedeutung haben,
   und
(2) Entfemen der Schutzgruppen von Z'.

Erfindungsgemäß wurde gefunden, daß die Verbindungen der allgemeinen Formeln (I) und (IV) durch phasentransferkatalysierte Umsetzung von Kohlenhydrathalogenosen zugänglich sind.

Bislang war nur aus O. Neunhoeffer, L. Lamza, Chem. Ber. 1961, 94, 2514-2521 die Synthese eines Triphenylphosphans bekannt, bei dem ein Phenylrest in p-Stellung durch einen Glucosylrest ersetzt ist, in dem die Hydroxylgruppen durch Acetylgruppen geschützt sind. Die Herstellung erfolgte durch Glycosidierung in wäßrigem Aceton mit Kaliumhydroxid als Base. Die Ausbeute betrug 8% und war somit wesentlich geringer als die im erfindungsgemäßen Verfahren erreichbaren Ausbeuten.

Die erfindungsgemäß hergestellten Phosphane weisen die allgemeine Formel (I) auf

Ar¹ ₃₋ₓP(Ar²(OZ)_{y})ₓ (I)

In der Formel (I) sind die Reste Ar¹ und Ar² aromatische C₆₋₂₀-Reste oder heteroaromatische C₄₋₉-Reste. Als aromatische Reste sind sie dabei vorzugsweise unabhängig Phenyl-, Naphthyl-, Biphenyl- oder Binaphthylreste liegen mehrere Reste Ar¹ bzw. Ar² vor, so kann jeder einzelne Rest unabhängig einer der vorstehenden Reste sein. Vorzugsweise ist dabei mindestens ein aromatischer Rest Ar¹ oder Ar² ein Phenylrest, besonders bevorzugt sind zwei der aromatischen Reste Phenylreste. Insbesondere sind alle aromatischen Reste Ar¹ und Ar² Phenylreste.

Als heteroaromatische Reste enthalten die Reste Ar¹ und/oder Ar² vorzugsweise 1 oder 2, besonders bevorzugt 1 Heteroatom, ausgewählt aus Sauerstoff, Schwefel und Stickstoff. Besonders bevorzugt sind auf Pyridin, Furan oder Thiophen basierende Reste. Die aromatischen Reste können jeweils unabhängig voneinander gegebenenfalls substituiert sein, beispielsweise mit Halogenatomen, Carboxylgruppen, Hydroxylgruppen, C₁₋₆-, vorzugsweise C₁₋₂-Alkylgruppen, Phenylgruppen oder Naphthylgruppen. Diese Substituenten können zusätzlich zu dem oder den Substituenten OZ des Restes Ar² vorliegen. Vorzugsweise sind die Substituenten Hydroxylgruppen, insbesondere liegt maximal eine Hydroxylgruppe pro aromatischem Kern vor.

Ar¹ besitzt eine und Ar² besitzt y plus 1 freie Valenzen. y hat dabei den Wert 1 oder 2. Ist der Rest Ar² ein auf einem Phenylrest basierender Rest, so können bei y = 1 die freien Valenzen in o-, m- oder p- Stellung vorliegen. Vorzugsweise liegen die freien Valenzen in o- oder p-, insbesondere in p-Stellung vor. Bei Biphenylresten oder Binaphthylresten als Reste Ar² liegen die Valenzen vorzugsweise an unterschiedlichen aromatischen Kernen vor, d.h. jeder Phenyloder Naphthylrest enthält vorzugsweise mindestens eine der freien Valenzen. y hat vorzugsweise den Wert 1.

x kann den Wert 1, 2 oder 3 haben. Vorzugsweise hat x den Wert 1 oder 2, insbesondere den Wert 1. y hat vorzugsweise den Wert 1.

Bevorzugte Verbindungen der Formel (I) sind solche, in denen y = 1 und x = 1 oder 2. Dabei ist der Rest Ar¹ vorzugsweise ein Phenylrest oder ein in p-Position hydroxylsubstituierter Phenylrest. Der Rest Ar² ist vorzugsweise ein p-Phenylenrest oder ein Binaphthylrest, insbesondere ein 1,1'-Binaphthylrest in dem die freien Valenzen in 2,2'-Stellung vorliegen.

Der Rest Z ist ein glycosidisch gebundener Kohlenhydratrest, insbesondere ein glycosidisch gebundener Rest, der sich von einem Zuckerrest ableitet. Vorzugsweise leitet sich Z von Glucose, Mannose, Galactose, Fructose, Cellobiose, Saccharose, Glucosamin, N-Acetylglucosamin oder deren Stereoisomeren ab. Z ist somit vorzugsweise von einem glycosidisch verknüpften 5- oder 6-gliedrigen Kohlenhydrat abgeleitet. Entsprechende Amine oder N-Acetylamine dieser Verbindungen können ebenfalls eingesetzt werden. Jeder Rest Z hat unabhängig eine der vorstehenden Bedeutungen.

Vorzugsweise leitet sich Z von Glucose, Galactose oder N-Acetylglucosamin ab.

Die erfindungsgemäßen Phosphane der allgemeinen Formel (I) werden hergestellt durch Umsetzung von Kohlenhydrathalogenosen der allgemeinen Formel (II)

Z'-X (II)

Dabei ist Z' ein Rest Z, wie er vorstehend beschrieben wurde, mit dem Unterschied, daß die Hydroxylgruppen durch Schutzgruppen substituiert sind. Geeignete Schutzgruppen, die die Hydroxylgruppen während der Umsetzungen vor einer Reaktion schützen, sind dem Fachmann bekannt. Beispiele sind Acetyl-, Benzoyl-, Benzyl- oder Allylgruppen. Die Schutzgruppen zeichnen sich dadurch aus, daß sie insbesondere eine Reaktion der geschützten Hydroxylgruppen bei Glycosilierungsreaktionen verhindern. Bevorzugt werden Acetylschutzgruppen eingesetzt. Die Herstellung der Verbindungen Z'-X kann beispielsweise nach dem in R.R. Schmidt, Angew. Chem. 1986, 98, 213-236 beschriebenen Verfahren erfolgen. Dabei wird beispielsweise das entsprechende Monosaccharid mit einer Base und einem Acylierungsmittel und gegebenenfalls einem Acylierungskatalysator umgesetzt. Die so entstandene peracylierte Verbindung wird mit einer Säure HX, vorzugsweise Bromwasserstoffsäure in Eisessig umgesetzt. Nach Standardaufarbeitungsverfahren erhält man die geschützte Halogenose. Die Verbindungen der Formel Z'-X, in denen X Brom, Chlor oder Fluor, insbesondere Brom oder Chlor ist, werden mit einem Phosphan der allgemeinen Formel (III)

Ar¹ ₃₋ₓP(Ar²(OH)_{y})ₓ (III)

umgesetzt. Ar¹, Ar², x, y, haben dabei die vorstehend angegebene Bedeutung. Die Umsetzung erfolgt in einem mehrphasigen, insbesondere zweiphasigen Reaktionsmedium in Gegenwart einer Base und eines Phasentransferkatalysators. Dabei wird bei Umsetzungstemperaturen von 0 - 80°C, vorzugsweise 10 - 60°C, insbesondere 20 - 50°C gearbeitet. Als Reaktionsmedien können alle geeigneten zwei- oder mehrphasigen Reaktionsmedien verwendet werden. Vorzugsweise ist eine Phase des Reaktionsmediums eine wäßrige Phase, wobei in dieser Phase Wasser gegebenenfalls im Gemisch mit einem wasserlöslichen organischen Lösungsmittel vorliegen kann. Die zweite Phase ist eine kaum oder nicht mit Wasser mischbare organische Phase. Beispiele geeigneter Zweiphasenreaktionsmedien sind Dichlormethan/Wasser, Aceton/ Wasser, Toluol/Wasser, Toluol/Ethylenglykol, tert.-Butylmethylether/Wasser, Dichlormethan/Polyethylenglykol.

Geeignete Basen sind Alkali- und Erdalkalihydroxide, basische Amine und Salze schwacher Säuren. Basische Amine sind beispielsweise Pyridin, Tributylamin, Benzylamin, Triethylamin, Diisopropylethylamin. Salze schwacher Säuren sind beispielsweise Natriumacetat, Kaliumbenzoat, Natriumcarbonat, Natriumhydrogenphosphat. Bevorzugt werden Alkali- und Erdalkalihydroxide, wie NaOH, KOH, LiOH, Ca(OH)₂, Mg(OH)₂ eingesetzt, wobei NaOH und KOH besonders bevorzugt sind.

Als Phasentransferkatalysatoren geeignete Katalysatoren sind beispielsweise in Eckehard N. Dehmlow, Sigrid S. Dehmlow, Phase Transfer Catalysis 3. Auflage, VCH, Weinheim, New York, Basel, Cambridge, Tokyo (1993) beschrieben. Vorzugsweise werden Tetra-n-C₁₋₁₂-alkylammoniumsalze eingesetzt. Besonders bevorzugt sind dabei Tetra-n-butylammoniumsalze. Als Gegenionen können alle geeigneten anorganischen Gegenionen verwendet werden, beispielsweise Halogenide oder Hydrogensulfate. Besonders bevorzugte Beispiele sind Tetra-nbutylammoniumbromid, Tetra-n-butylammoniumhydrogensulfat, Tetra-nbutylammoniumchlorid, Tetra-n-butylammoniumnitrat. Die Phasentransferkatalysatoren werden vorzugsweise in einer Menge von 1 bis 90 Gew.-%, bezogen auf Halogenose eingesetzt.

Zur Darstellung von N-Acetylglucosamin-Verbindungen wird vorzugsweise wie folgt vorgegangen:

Verwendbare N-Acetylglucosamindonoren sind beispielsweise beschrieben in T Mukaiyama et al., Chem. Lett. 1984, 907; K. Higashai, Chem. Pharm. Bull. 1990, 38, 3280. Von den verschiedenen Glucosamindonoren ist das Glucopyranosylchlorid durch Umsetzung mit Acetylchlorid direkt in einer Stufe zugänglich. Dies ist beispielsweise beschrieben in D. Horton, Org. Synth., Coll. Vol. V, 1973, 1. Weiterhin kann nach dem in G. Chittenden, Carbohydr. Res. 1993, 242, 297 beschriebenen Verfahren vorgegangen werden. Als Phasentransferkatalysator für die Kupplungsreaktion wird vorzugsweise Zinkchlorid eingesetzt, das eine gute alpha/beta-Selektivität ergibt (T. Norberg, J. Carbohydr. Chem. 1990, 9, 721). Vorzugsweise erfolgt die Umsetzung in Gegenwart von Co-Katalysatoren. Geeignete Co-Katalysatoren sind beispielsweise in R. Bittman, Tetrahedron Lett., 1994, 35, 505 beschrieben. Vorzugsweise wird statt dem gemäß in dieser Literaturstelle verwendeten Tritylchlorid ein 4,4'-Dimethoxydtritylhalogenid in Kombination mit Zinkfluorid eingesetzt. Eine Epimerisierung konnte damit verhindert werden, zudem erfolgt die Umsetzung leichter. Beim Einsatz dieser Katalysatorkombination konnte so eine deutliche Verkürzung der Reaktionszeit auch bei größeren Reaktionsansätzen erreicht werden. Somit wird zur Darstellung von N-Acetylglucosaminderivaten besonders bevorzugt die Katalysatorkombination Zinkchlorid/4,4'-Dimethoxytritylhalogenid als Phasentransferkatalysator eingesetzt. Bei Verwendung dieses Katalysatorsystems ist die chromatographische Reinigung der Produkte zudem vereinfacht. Die kürzere Reaktionszeit ermöglicht die gezielte Synthese von beta-glycosidisch verknüpften N-Acetylglucosaminderivaten in guten Ausbeuten und ohne auftretende Epimerisierung.

Nach der Umsetzung zu Phosphanen der allgemeinen Formel (IV) werden die Schutzgruppen von Z' entfernt. Geeignete Verfahren sind beispielsweise beschrieben in R.R. Schmidt, Angew. Chem. 1986, 98, 213-236. Vorzugsweise erfolgt die Abspaltung von Acetylschutzgruppen unter basischen Bedingungen.

Das erfindungsgemäße Verfahren ermöglicht die Herstellung einer Vielzahl von Kohlenhydrat-substituierten Triphenylphosphanen in kurzen Reaktionszeiten und mit hohen Ausbeuten.

Die Erfindung betrifft auch Phosphane der allgemeinen Formel (I). wie sie vorstehend definiert sind. Zudem betrifft die Erfindung Phosphane der allgemeinen Formel (IV). wie sie vorstehend definiert sind. Ausgenommen sind Verbindungen mit Ar¹ = Phenyl. Ar² = p-Phenyien. Z'= acetylgeschützter Glucosylrest, x = 1, y = 1.

Beispiele bevorzugter Verbindungen der allgemeinen Formel (IV) sind die nachstehenden:
Diphenyl-(o-hydroxyphenyl)-phosphin-2,3,4,6-tetra-O-acetyl-β-D-glucopyranosid, 1-O-[4-(Diphenylphosphino)phenyl]-2-acetamido-2-desoxy-3,4,6-O- acetyl-β-D-glucopyranosid.

Bevorzugte Phosphane der allgemeinen Formel (I) entsprechen diesen Verbindungen, wobei die Schutzgruppen entfernt sind.

Die erfindungsgemäßen Phosphane der allgemeinen Formel (I) können zur Herstellung von Metallkomplexen verwendet werden. Dabei können als zentrales Metallatom folgende Metalle eingesetzt werden: Ru, Pd, Rh, Ni, Pt, Co, Ir, Cu, Fe, Mn. Die Metallkomplexe können neben den erfindungsgemäßen Phosphanen der allgemeinen Formel (I) als Liganden weitere Liganden, wie Halogenatome, Wasserstoffatome, Kohlenmonoxid u.s.w. enthalten.

Die Erfindung betrifft auch Rutheniumkomplexe der allgemeinen Formel (V)

RuH₂(CO)L₃ (V)

in der L ein Phosphan der allgemeinen Formel (I) ist, wie es vorstehend beschrieben ist. Die Komplexe werden durch Umsetzung des Phosphans mit Ruthenium(III)-chloridtrihydrat nach dem in J.J. Levison, S.D. Robinson, J. Chem. Soc. A 1970, 2947-2954 beschriebenen Verfahren hergestellt.

Die Erfindung betrifft auch Palladiumkomplexe der allgemeinen Formel (VI)

L₂PdX₂ (VI)

in der L ein Phosphan der allgemeinen Formel (I) wie vorstehend beschrieben und X Cl, Br, I, vorzugsweise Cl, oder Acetat ist. Die Herstellung erfolgt z.B. mit Lithiumtetrachloropalladat(II) in Ethanol nach dem in G. Brauer, Handbuch der präparativen anorganischen Chemie, Ferdinand Enkel Verlag, 1981, Band 3, Seite 2014 beschriebenen Verfahren.

Die Komplexe, in denen X Acetat ist, können in situ aus Pd(OAc)₂ und dem entsprechenden Liganden im Reaktionsgemisch erzeugt werden. Das Verhältnis von Pd zu Ligand beträgt dabei vorzugsweise 1:1 bis 1 :20, besonders bevorzugt 1:2 bis 1:10, insbesondere etwa 1:3. Unter reduzierenden Bedingungen entstehen daraus Palladiumkomplexe der allgemeinen Formel (VII)

L_{z}Pd (VII)

In der L ein Phosphan der allgemeinen Formel (I) ist und z den Wert 3 oder 4 hat.

Die erfindungsgemäßen Katalysatoren können in katalytischen C-C-Kupplungsreaktionen eingesetzt werden. Beispiele solcher Kupplungsreaktionen sind die Heck-Reaktion, die Suzuki-Reaktion oder Hydroformylierungen, weiterhin sind sie für Hydrierungen von ungesättigten Verbindungen, Hydrierungen von Nitroaromaten, Allylsubstitutionen, Carbonylierungen und Polymerisationen verwendbar.

Nachstehend wird die Erfindung anhand von Beispielen näher erläutert:

### Beispiel 1:

### 1-O-[4-(Diphenylphosphino)phenyl]-2-acetamido-2-desoxy-β-D-glucopyranosid (Glycosidierung)

202 mg (0.726 mmol) (p-Hydroxyphenyl)diphenylphosphan und 125 mg (0.368 mmol) Tetra-n-butylammoniumhydrogensulfat werden in 2 ml Methylenchlorid gelöst und mit 2 ml 1 M Natronlauge versetzt. Es werden 133 mg (0.364 mmol) 2-Acetamido-3,4,6-tri-O-acetyl-2-desoxy-α-D-glucopyranosylchlorid (1) und nach 5 min. weitere 133 mg (0.364 mmol) 1 zugegeben. Nach 15 min werden 0.5 ml 1 M Natronlauge und 267 mg (0.730 mmol) 1, nach 30 min 1 ml Methylenchlorid, 0.5 ml 1 M Natronlauge und 262 mg (0.716 mmol) 1, nach 50 min 0.5 ml 1 M Natronlauge und 262 mg (0.716 mmol) 1 und schließlich nach 70 min 0.5 ml 1 M Natronlauge und 259 mg (0.708 mmol) 1 zugegeben. Nach 1.5 h wird mit 30 ml Essigester verdünnt. Die organische Phase wird zweimal mit je 20 ml 1 M Natronlauge, zweimal mit je 20 ml Wasser und einmal mit 15 ml gesättigter Kochsalzlösung gewaschen. Es wird über Magnesiumsulfat getrocknet und die Lösungsmittel werden abdestilliert. Der Rückstand wird in 10 ml absolutem Methanol aufgenommen und unter Stickstoff mit 0.5 ml einer 1 M methanolischen Natriummethanolat-Lösung versetzt. Nach 30 min Rühren bei Raumtemperatur wird mit 10 ml Methanol verdünnt und mit Amberlyst H 15 (stark saurer Kationenaustauscher) neutralisiert. Der lonenaustauscher wird abfiltriert und die Lösungsmittel abdestilliert. Der Rückstand wird mit Chloroform/Methanol/Hexan 6:1:1 (v/v/v) über 55 g Kieselgel 60 chromatographiert. - Ausbeute: 238 mg (68% bezgl. Phosphan).

### 1-O-[4-(Diphenylphosphino)phenyl]-2-acetamido-2-desoxy-β-D-glucopyranosid (Entschützung)

0,33 mmol 1-O-[4-(Diphenylphosphino)phenyl]-2-acetamido-2-desoxy-3,4,6-Oacetyl-β-D-glucopyranosid werden in 25 ml Methanol suspendiert und eine Lösung von 2 mg Natrium in 25 ml Methanol zugegeben. Nach ca. 2 Stunden Rühren bei RT ist eine Lösung entstanden. Man neutralisiert mit saurem Ionenaustauscher (Amberlyst H 15), filtriert und engt den Rückstand bis zur Trockene ein. Der Rückstand wird mit Wasser gewaschen und aus Ethanol/Wasser umkristallisiert. - Ausbeute: 0,32 mmol (96%).

### Beispiel 2:

Diphenyl-(p-Hydroxyphenyl)-phosphin-2,3,4,6-tetra-O-acetyl-β-D-glucopyranosid Zu einer Lösung von Diphenyl-(p-hydroxyphenyl)-phosphin (778 mg, 2,80 mmol) in Wasser/Aceton (10 ml/6 ml) wird Kaliumhydroxid (0.35 g) gegeben. Unter Rühren wird eine Lösung von Acetobromglucose (2.63 g, 6.4 mmol) in Aceton (15 ml) zugetropft. Das Gemisch wird 2 d bei Raumtemperatur gerührt. Nach Entfernen des Lösungsmittels am Rotationsverdampfer wird der Rückstand mehrfach mit Wasser gespült. Der verbleibende Rückstand wird aus Ethanol umkristallisiert. Man erhält das Glucopyranosid als leicht gelben Feststoff (450 mg, 26,4%).
R_{f}-Wert (Toluol/Essigester 1/1): 0,61
¹H-NMR (300 MHz, DMSO):
7,45-7,18 (m 12 H); 7,07-7,01 (m, 2H), 5,62 (d, J=8,0 Hz, 1H); 5,40 (m, J=8,8 Hz, 1H), 5,06 (m, J=9,7 Hz, J=8.2 Hz, 1H); 5,00 (m, J=9,7 Hz, 1H); 4,28-4,04 (m, 3H); 2,02-1,95 (4s, jeweils 3H).

### Diphenyl-(p-hydroxyphenyl)-phosphin-β-D-glucopyranosid

Diphenyl-(p-hydroxyphenyl-phosphin-2,3,4,6-tetra-O-acetyl-β-D-glucopyranosid (196 mg, 0,32 mmol) wird in absolutem Methanol (5 ml) gelöst und mit 30%iger Natriummethanolat-Lösung (0,5 ml) versetzt. Die Lösung wird 3 h bei Raumtemperatur gerührt und mit saurem Ionenaustauscher (Dowex 50 W X 8) neutralisiert. Nach Entfernen des Lösungsmittels erhält man das Glycopyranosid als leicht gelben Feststoff (139 mg. 97%).
R_{f}-Wert (Dichlormethan/Methanol 10/1): 0,3

### Beispiel 3

Diphenyl-(o-hydroxyphenyl)-phosphin-2,3,4,6-tetra-O-acetyl-β-D-glucopyranosid Zu einer Lösung von Diphenyl-(o-hydroxyphenyl)-phosphin (4,33 g, 15,6 mmol) in Wasser/Aceton (60 ml/35 ml) wird Kaliumhydroxid (1,91 g) gegeben. Unter Rühren wird eine Lösung von Acetobromglucose (14,25 g) in Aceton (15 ml) zugetropft. Das Gemisch wird 2 d bei Raumtemperatur gerührt. Nach Entfernen des Lösungsmittels am Rotationsverdampfer wird der Rückstand mehrfach mit Wasser gespült. Der verbleibende Rückstand wird aus Ethanol umkristallisiert. Man erhält das Glycopyranosid als leicht gelben Feststoff (1,84 g, 19,4%).
R_{f}-Wert (Toluol/Essigester 1/1):0,6
¹H-NMR (300 MHz, DMSO):
7,50-7,00 (m, 14H); 5,62 (d, J=8,1 Hz, 1H); 5,42 (m, J=8,7 Hz, 1H), 5,10 (m, J=9,7 Hz, J=8.1 Hz, 1H); 5,00 (m, J=9,7 Hz, 1H); 4,28-4,06 (m, 3H); 2,03-1,95 (4s, jeweils 3H).

### Diphenyl-(o-hydroxyphenyl)-phosphin-β-D-glucopyranosid

Diphenyl-(o-hydroxyphenyl)-phosphin-2,3,4,6-tetra-O-acetyl-β-D-glycopyranosid (450 mg, 0.74 mmol) wird in absolutem Methanol (5 ml) gelöst und mit 30%iger Natriummethanolat-Lösung 0.5 ml) versetzt. Die Lösung wird 3 h bei Raumtemperatur gerührt und mit saurem Ionenaustauscher (Dowex 50 W X 8) neutralisiert. Nach Entfernen des Lösungsmittels erhält man das Glycopyranosid als leicht gelben Feststoff (312 mg, 96%).
R_{f}-Wert (Dichlormethan/Methanol 10/1): 0,4

### Beispiel 4

### Darstellung von GlcNAc-Chlorid

Zu 500 ml Acetylchlorid werden innerhalb von 2 min 250 g (1,13 mol) N-Acetyl-(D)-glucosamin gegeben. Das Gemisch wird 19 h bei Raumtemperatur gerührt. Nach Zugabe von Methylenchlorid (500 ml) wird die Lösung auf ein Eis/Wasser Gemisch (500 ml) gegeben und die organische Phase schnell abgetrennt. Die organische Phase wird schnell mit gesättigter Natriumhydrogencarbonatlösung gewaschen, über Magnesiumsulfat getrocknet und filtriert. Das Gemisch wird auf die Hälfte des Volumens am Rotationsverdampfer eingeengt. Unter schnellem Rühren werden 500 ml Diethylether zugefügt. Nach 12 h wird der entstandene Niederschlag abgesaugt und unter vermindertem Druck getrocknet. Man erhält das Chlorid als leicht braunes Pulver (269 g, 62%).

### Diphenyl-(o-hydroxyphenyl)-phosphin-N-acetyl-2-amino-2-desoxy-3,4,6-tri-O-acetyl-β-D-glycopyranosid

Zu einer Suspension von Zink(II)-chlorid (139 mg, 1,03 mmol) und 4,4'-Dimethoxytritylchlorid (122 mg, 1,34 mmol) in trockenem Dichlormethan (15 ml) werden das Chlorid (1.3 eq, 497 mg, 1,34 mmol) und Diphenyl-(ohydroxyphenyl)-phosphin (287 mg, 1,34 mmol) gegeben. Das Gemisch wurde 13 h bei Raumtemperatur gerührt (DC Kontrolle Methylenchlorid/Methanol 50/1). Nach Zugabe von Methylenchlorid wird mit ges. Natriumhydrogencarbonatlösung gewaschen. Nach Entfernen des Lösungsmittels am Rotationsverdampfer wird der Rückstand säulenchromatographisch (Methylenchlorid/ Methanol 100/1-40/1 gereinigt). Man erhält das Glycosid in 37% Ausbeute (232 mg).
R_{f}-Wert (Dichlormethan/Methanol 50/1): 0,4
¹H-NMR (300 MHz, DMSO):
7,51-7,01 (m, 14 H); 5,60 (d, J=8,2 Hz, 1H); 5,40 (m, J=8,6 Hz, 1H), 5,15 (m, J=9,7 Hz, J=8,2 Hz, 1H); 5,03 (m, J=9,6 Hz, 1H); 4,28-4,02 (m, 3H); 2,17-1,95 (4s, jeweils 3H).

### Katalytische Umsetzungen

### Beispiel 5

### Suzuki-Reaktion

4-Bromacetophenon oder 1-Brom-4-chlorbenzol wurden gemäß der Suzuki-Reaktion mit Phenylboronsäure (H₅C₆-B(OH)₂) zu den entsprechend substituierten Biphenylen umgesetzt. Dabei wurde das Bromatom durch den Phenylrest ersetzt. Ein Gemisch aus 15 mmol Phenylboronsäure, 13,5 mmol 4-Bromacetophenon oder 1-Brom-4-chlorbenzol, 40,5 mmol Na₂CO₃ 10 H₂O, 0,01 Mol-%, bezogen auf Phenylboronsäure, Pd(OAc)₂, und Ligand L in einem Molverhältnis Pd:L von 1:3 wurden in einem Lösungsmittelgemisch aus 9 ml Ethanol, 9 ml Wasser und 18 ml Di-n-butylether beziehungsweise 12 ml Ethanol, 6 ml Wasser und 15 ml Toluol unter Rühren auf eine Temperatur von 60°C erhitzt. Sodann wurde das Gemisch für 2 Stunden auf eine Temperatur von 78°C erhitzt. Das Umsetzungsprodukt wurde isoliert und die Ausbeute bestimmt. Als Ligand wurde eingesetzt. Zu Vergleichszwecken wurde als Ligand das Trinatriumsalz der 3,3',3"- Phosphantriylbenzolsulfonsäure (TPPTS) eingesetzt. Die Ergebnisse der Umsetzung sind in der nachstehenden Tabelle 1 für die Umsetzung mit 4-Bromacetophenon angegeben.

**Tabelle 1**

| Versuch | Ligand | Ausbeute [%] | Lösungsmittel | TON |
|---|---|---|---|---|
| 1 | 3a | 87 | 1 | 8700 |
| 2 | TPPTS* | 67 | 1 | 6700 |
| 3 | 3b | 90 | 2 | 9000 |
| 4 | TPPTS* | 87 | 2 | 8700 |

| | | | | |
|---|---|---|---|---|
| *TPPTS = Trinatriumsalz der 3,3',3"-Phosphantriylbenzolsulfonsäure, Vergleichsligand | | | | |

Lösungsmittel 1: 9 ml Ethanol, 9 ml Wasser, 18 ml Di-n-butylether
Lösungsmittel 2: 12 ml Ethanol, 6 ml Wasser, 15 ml Toluol
TON: Umsatzzahl = mol(Produkt)/mol(Katalysator).

Für die Umsetzung mit 1-Brom-4-chlorbenzol sind die Ergebnisse in Tabelle 2 angegeben.

**Tabelle 2**

| Versuch | Ligand | Ausbeute [%] | Lösungsmittel | TON |
|---|---|---|---|---|
| 5 | 3a | 56 | 1 | 5600 |
| 6 | TPPTS* | 40 | 1 | 4000 |
| 7 | 3b | 71 | 2 | 7100 |
| 8 | TPPTS* | 44 | 2 | 4400 |

Aus den Ergebnissen aus Tabellen 1 und 2 geht hervor, daß Ausbeuten und Umsätze mit Katalysatoren, die die erfindungsgemäßen Liganden aufweisen, wesentlich höher sind als bei Verwendung des Vergleichsliganden TPPTS.

### Beispiel 6

### Heck-Reaktion

In der zweiphasig ausgeführten Heck-Reaktion wurden 4-Bromacetophenon beziehungsweise 1-Brom-4-nitrobenzol mit Styrol zu den entsprechenden substituierten Stilbenen umgesetzt. Dabei wurde ein Gemisch aus 15 mmol 4-Bromacethopenon oder 1-Brom-4-nitrobenzol, 22,5 mmol (1,5 Äquivalente) Styrol, 16,5 mmol (1,1 Äquivalente) NaOAc 3 H₂O, Pd(OAc)₂ in der nachstehenden Tabelle 3 angegebene Menge und die in der Tabelle angegebenen Liganden im Verhältnis Pd:L = 1:3 in einem Gemisch aus 10 ml Xylol und 10 ml Ethylenglykol unter Rühren bei 130°C für 20 Stunden umgesetzt. Das Umsetzungsprodukt wurde isoliert und seine Ausbeute bestimmt. Die Ergebnisse sind in den nachstehenden Tabellen 3 und 4 zusammengefaßt:

**Tabelle 3:**

| Heck-Reaktion mit 4-Bromacetophenon | | | | |
|---|---|---|---|---|
| Versuch | Ligand | Ausbeute [%] (E:Z) | Katalysator Mol [%] | TON |
| 9 | 3a | 98 (89:11) | 1 | 98 |
| 10 | 3b | 80 (95:5) | 1 | 80 |
| 11 | TPPTS* | 79 (92:8) | 1 | 79 |

**Tabelle 4:**

| Heck-Reaktion mit 1-Brom-4-nitrobenzol | | | | |
|---|---|---|---|---|
| Versuch | Ligand | Ausbeute [%] (E:Z) | Katalysator Mol [%l | TON |
| 12 | 3a | 85 (95:5) | 0,1 | 850 |
| 13 | 3b | 88 (95:5) | 0,1 | 880 |
| 14 | TPPTS* | 78 (94:6) | 1 | 78 |

Es wird auf die Erläuterungen der ,Tabelle 1 verwiesen. Die Katalysatoren mit den erfindungsgemäßen Liganden zeigen hohe Ausbeuten und hohe Umsatzzahlen bei einem großen Verhältnis von E- zu Z-Isomer.

## Patentansprüche

1. Verfahren zur Herstellung von Phosphanen der allgemeinen Formel (I)
Ar¹ ₃₋ₓP(Ar²(OZ)_{y})ₓ (I)
in der Ar¹ und Ar² unabhängig gegebenenfalls mit Halogenatomen, Carboxylgruppen, Hydroxylgruppen, C₁₋₆-Alkylgruppen, Phenylgruppen oder Naphthylgruppen substituierte aromatische C₆₋₂₀-Reste oder heteroaromatische C₄₋₉-Reste sind, wobei Ar¹ eine und Ar² y + 1 freie Valenzen besitzen,
Z ein glycosidisch gebundener Kohlenhydratrest ist,
x den Wert 1, 2 oder 3 und
y den Wert 1 oder 2 hat,
durch
(1) Umsetzung von Kohlenhydrathalogenosen der allgemeinen Formel (II)
Z'-X (II)
in der Z' ein wie vorstehend angegebener Rest Z ist, in dem die Hydroxylgruppen durch Schutzgruppen substituiert sind, und X Br, Cl, oder F ist,
mit einem Phosphan der allgemeinen Formel (III)
Ar¹ ₃₋ₓP(Ar²(OH)_{y})ₓ (III)
in der Ar¹, Ar², x, y die vorstehende Bedeutung haben, in einem mehrphasigen Reaktionsmedium in Gegenwart einer Base und eines Phasentransferkatalysators,
zu einem Phosphan der allgemeinen Formel (IV)
Ar¹ ₃₋ₓP(Ar²(OZ')_{y})ₓ (IV)
in der Ar¹, Ar², x, y und Z' die vorstehend angegebene Bedeutung haben,
und
(2) Entfernen der Schutzgruppen von Z'.

2. Verfahren nach Anspruch 1, wobei Ar¹ und Ar² unabhängig Phenyl-, Naphthyl-, Biphenyl-, Binaphthyl-, oder Pyridylreste sind.

3. Verfahren nach Anspruch 1 oder 2, wobei sich Z und Z' von Glucose, Mannose, Galactose, Fructose, Cellobiose, Saccharose, Glucosamin oder N-Acetylglucosamin oder deren Stereoisomeren ableiten.

4. Verfahren nach einem der vorstehenden Ansprüche, wobei die Schutzgruppen Acetyl-, Benzoyl,- Benzyl- oder Allylgruppen sind.

5. Phosphane der allgemeinen Formel (I), wie sie in einem der Ansprüche 1 bis 3 definiert sind, ausgenommen die Verbindung mit Ar¹ = Phenyl, Ar² = p-Phenylen, Z = β-Glucosyl, x = 1, y = 1.

6. Phosphane der allgemeinen Formel (IV) wie sie in einem der Ansprüche 1 bis 4 definiert sind, ausgenommen die Verbindung mit Ar¹ = Phenyl, Ar² = p-Phenylen, Z' = acetylgeschütztes Glucosyl, x = 1, y = 1.

7. Verwendung von Phosphanen nach Anspruch 5 oder 6 zur Herstellung von Metallkomplexen.

8. Rutheniumkomplexe der allgemeinen Formel (V)
RuH₂(CO)L₃ (V)
in der L ein Phosphan der allgemeinen Formel (I) nach Anspruch 5 ist.

9. Palladiumkomplexe der allgemeinen Formel (VI)
L₂PdX₂ (VI)
in der L ein Phosphan der allgemeinen Formel (I) nach Anspruch 5 und X Cl, Br, I oder Acetat ist.

10. Palladiumkomplexe der allgemeinen Formel (VII)
L_{z}Pd (VII)
in der L ein Phosphan der allgemeinen Formel (I) nach Anspruch 1 ist und z den Wert 3 oder 4 hat.

11. Verwendung von Komplexen nach einem der Ansprüche 8 bis 10 als Katalysatoren in katalytischen C-C-Kupplungsreaktionen, insbesondere Heckreaktionen, Suzukireaktionen oder Hydroformylierungen.

## Claims

1. Process for the production of phosphanes having the general formula (I)
Ar¹ ₃₋ₓP(Ar²(OZ)_{y})ₓ (I)
in which Ar¹ and Ar² independently are aromatic C₆₋₂₀ radicals or heteroaromatic C₄₋₉ radicals substituted, if appropriate, by halogen atoms, carboxyl groups, hydroxyl groups, C₁₋₆ alkyl groups, phenyl groups or naphthyl groups wherein Ar¹ has one and Ar² has y + 1 free valences,
Z is a carbohydrate radial with a glycosidic bond,
x has the value 1, 2 or 3, and
y has the value 1 or 2,
by
(1) the conversion of hydrocarbon halogenoses having the general formula (II)
Z'-X (II)
in which Z' is a radical Z as specified above, in which the hydroxyl groups are substituted by protective groups, and X represents Br, Cl or F, with a phosphane having the general formula (III)
Ar¹ ₃₋ₓP(Ar²(OH)_{y})ₓ (III)
in which Ar¹, Ar², x and y have the above meaning
in a multiphase reaction medium in the presence of a base and of a phase transfer catalyst,
into a phosphane having the general formula (IV)
Ar¹ ₃₋ₓP(Ar²(OZ')_{y})ₓ (IV)
in which Ar¹, Ar², x, y and Z' have the above specified meaning, and
(2) removal of the protective groups from Z'.

2. Process as claimed in claim 1 in which Ar¹ and Ar² independently are phenyl, naphthyl, biphenyl, binaphthyl or pyridyl radicals.

3. Process as claimed in claim 1 or 2, in which Z and Z' are derived from glucose, mannose, galactose, fructose, cellobiose, saccharose, glucosamine or N-acetyl glucosamine or their stereoisomers.

4. Process as claimed in one of the above claims, in which the protective groups are acetyl, benzoyl, benzyl or allyl groups.

5. Phosphanes having the general formula (I) such as are defined in one of claims 1 to 3, with the exception of the compound in which Ar¹ = phenyl, Ar² = p-phenylene, Z = β-glucosyl, x = 1, y = 1.

6. Phosphanes having the general formula (IV) such as are defined in one of claims 1 to 4, with the exception of the compound in which Ar¹ = phenyl, Ar² = p-phenylene, Z' = acetyl-protected glucosyl, x= 1, Y = 1.

7. Use of phosphanes as claimed in claim 5 or 6 for the production metal complexes.

8. Ruthenium complexes having the general formula (V)
RuH₂(CO)L₃ (V)
in which L is a phosphane having the general formula (I) as claimed in claim 5.

9. Palladium complexes having the general formula (VI)
L₂PdX₂ (VI)
in which L is a phosphane having the general formula (I) as claimed in claim 5 and X is Cl, Br,I or acetate.

10. Palladium complexes having the general formula (VII)
L_{z}Pd (VII)
in which L is a phosphane having the general formula (I) as claimed in claim 1 and z has the value 3 or 4.

11. Use of complexes as claimed in one of claims 8 to 10 as catalysts in catalytic C-C coupling reactions, in particular Heck reactions, Suzuki reactions or hydroformylations.

## Revendications

1. Procédé pour la préparation de phosphanes de formule générale (I)
Ar¹ ₃₋ₓP(Ar²(OZ)_{y})ₓ (I)
dans laquelle Ar¹ et Ar² représentent, indépendamment, des radicaux aromatiques en C₆ à C₂₀ ou des radicaux hétéroaromatiques en C₄ à C₉, le cas échéant substitués par des atomes d'halogène, des groupes carboxyle, des groupes hydroxyle, des groupes alkyle en C₁ à C₆, des groupes phényle ou des groupes naphtyle, Ar¹ étant monovalent et Ar² présentant y+1 valences,
Z représente un radical hydrate de carbone lié glycosidiquement,
x vaut 1, 2 ou 3 et
y vaut 1 ou 2,
par
(I) transformation d'halogénoses d'hydrates de carbone de formule générale (II)
Z'-X (II)
dans laquelle Z' représente, comme indiqué ci-dessus, un radical Z dans lequel les groupes hydroxyles sont substitués par des groupes de protection et X représente Br, Cl ou F,
avec un phosphane de formule générale (III)
Ar¹ ₃₋ₓP(Ar²(OH)_{y})ₓ (III)
dans laquelle Ar¹, Ar², x, y ont la signification susmentionnée, dans un mélange réactionnel à plusieurs phases, en présence d'une base et d'un catalyseur de transfert de phase,
en un phosphane de formule générale (IV)
Ar¹ ₃₋ₓP(Ar²(OZ')_{y})ₓ (IV)
dans laquelle Ar¹, Ar², x, y et Z' ont la signification susmentionnée et
(2) élimination des groupes de protection de Z'.

2. Procédé selon la revendication 1, dans lequel Ar¹ et Ar² représentent, indépendamment, des radicaux phényle, naphtyle, biphényle, binaphtyle ou pyridyle.

3. Procédé selon la revendication 1 ou 2, dans lequel Z et Z' sont dérivés du glucose, du mannose, du galactose, du fructose, de la cellobiose, du saccharose, de la glucosamine ou de la N-acétylglucosamine ou leurs stéréoisomères.

4. Procédé selon l'une quelconque des revendications précédentes, dans lequel les groupes de protection sont des groupes acétyle, benzoyle, benzyle ou allyle.

5. Phosphanes de formule générale (I), tels qu'ils sont définis dans l'une quelconque des revendications 1 à 3, à l'exception du composé avec Ar¹ = phényle, Ar² = p-phénylène, Z = β-glucosyle, x = 1, y = 1.

6. Phosphanes de formule générale (IV) tels qu'ils sont définis dans l'une quelconque des revendications 1 à 4, à l'exception du composé avec Ar¹ = phényle, Ar² = p-phénylène, Z'= glucosyle protégé par acétyle, x = 1, y = 1.

7. Utilisation de phosphanes selon la revendication 5 ou 6 pour la préparation de complexes métalliques.

8. Complexes du ruthénium de formule générale (V)
RuH₂(CO)L₃ (V)
dans laquelle L est un phosphane de formule générale (I) selon la revendication 5.

9. Complexes du palladium de formule générale (VI)
L₂PdX₂ (VI)
dans laquelle L est un phosphane de formule générale (I) selon la revendication 5 et X représente Cl, Br, I ou acétate.

10. Complexes du palladium de formule générale (VII)
L_{z}Pd (VII)
dans laquelle L est un phosphane de formule générale (I) selon la revendication 1 et z vaut 3 ou 4.

11. Utilisation de complexes selon l'une quelconque des revendications 8 à 10 comme catalyseurs dans des réactions catalytiques de couplage C-C, en particulier des réactions de Heck, de Suzuki ou des hydroformylations.
